Europäisches Patentamt

European Patent Office

Office européen des brevets

(11) Veröffentlichungsnummer: **0 426 856 A1**

(12)

# EUROPÄISCHE PATENTANMELDUNG
## veröffentlicht nach Art. 158 Abs. 3 EPÜ

㉑ Anmeldenummer: 90902067.9

㉒ Anmeldetag: 16.10.89

㊻ Internationale Anmeldenummer:
PCT/SU89/00266

㊼ Internationale Veröffentlichungsnummer:
WO 90/12613 (01.11.90 90/25)

㉛ Int. Cl.⁵: **A61M 5/50**

㉚ Priorität: 24.04.89 SU 4682849

㊸ Veröffentlichungstag der Anmeldung:
**15.05.91 Patentblatt 91/20**

㊱ Benannte Vertragsstaaten:
**BE CH DE FR GB IT LI SE**

㉛ Anmelder: **POLTAVSKY MEDITSINSKY
STOMATOLOGICHESKY INSTITUT
ul. Shevchenko, 23,
Poltava 314000(SU)**

㉒ Erfinder: **MAZURIK, Sergei Mikhailovich
ul. Lenina, 92-57
Poltava, 314022(SU)**
Erfinder: **SOKOLOV, Andrei Nikolaevich
ul. 60 let SSSR, 3-20
Poltava, 314022(SU)**

㊔ Vertreter: **Nix, Frank Arnold, Dr.
Kröckelbergstrasse 15
W-6200 Wiesbaden(DE)**

(54) **EINWEGINJEKTIONSSPRITZE.**

㊗ Die Erfindung bezieht sich auf die medizinische Technik.

Die Einmalspritze für Injektionen besteht aus einem Zylinder (1) und einem in diesem angeordneten Kolben (2) mit Kolbenstange (3). Die Kolbenstange (3) ist profiliert ausgeführt, und zwar in Gestalt einer Vielzahl von längs der Kolbenstange aufeinanderfolgenden Kegelstümpfe (7), und konzentrisch zu ihr ist ein hohler profilierter Einsatz (8) angeordnet, der aus zwei Teilen (8a, 8b) besteht, dessen Innenfläche der Außenfläche der Kolbenstange entspricht und dessen Außenfläche durch die Oberflächen einer Vielzahl von Kegelstümpfen (9) gebildet ist. Zwischen dem Kolben (2) und dem Einsatz (8) ist konzentrisch zur Kolbenstange (3) eine Ringscheibe (10) angeordnet, deren seitliche Mantelfläche geneigt ausgebildet ist und an der Innenfläche des Zylinders (1) ist ein Ringbund (11) ausgebildet, dessen eine Oberfläche geneigt ausgebildet ist und der geneigten Oberfläche der Scheibe (10) entspricht.

Die Einmalspritze kann in beliebigen medizinischen Anstalten und zum privaten Gebrauch verwendet werden.

FIG.8

## EINMALSPRITZE FÜR INJEKTIONEN

Technisches Gebiet

Die vorliegende Erfindung bezieht sich auf das Gebiet medizinischer Ausrüstungen, insbesondere auf Einmalspritzen für Injektionen.

Mit Rücksicht auf die weite Verbreitung der AIDS-Epidemie in der ganzen Welt, für die ein Hauptübertragungsweg Injektionen mit Spritzen und Nadeln sind, besteht das Problem der Schaffung zuverlässiger Einmalspritzen. Dabei muß die größte Aufmerksamkeit gerichtet sein auf das zuverlässige Ausschließen jeder Möglichkeit, eine Einmalspritze wiederholt zu verwenden.

Zugrundeliegender Stand der Technik

Allgemein bekannt sind zur Zeit Einmalspritzen für Injektionen, die einen Zylinder, einen in diesem untergebrachten Kolben mit seiner Stange, eine Öffnung an dem hinteren Stirnende des Zylinders zum Durchgang der Kolbenstange und eine Vorrichtung zum Befestigen der Nadel am vorderen Stirnende des Zylinders einschließen (beispielsweise die Einmalspritze aus der Produktion der Firma "Trumo Europe", Belgien). Die genannten Spritzen unterscheiden sich von den in der medizinischen Praxis weitgehend bekannten Spritzen zur mehrmaligen Verwendung praktisch in nichts mit nur der Ausnahme, daß sie aus einem billigeren (polymeren) Werkstoff bestehen und nicht sterilisiert werden. Somit erlaubt die Konstruktion der bekannten Einmalspritzen deren mehrmalige Verwendung. Diese wiederholte Verwendung kann auf Grund fehlender Aufmerksamkeit bzw. mangelnder Gewissenhaftigkeit einer medizinischen Fachkraft und bei der Durchführung der Injektionen von Personen im Alkohol- oder Rauschgiftrausch vorkommen. Diese Umstände sind deshalb wichtig, weil in diesen Fällen eine Ansteckung mit dem AIDS-Virus, mit einer infektiösen Hepatitis und anderen Erkrankungen möglich ist.

Bekannt ist auch eine andere Einmalspritze, die einen Zylinder, einen in diesem untergebrachten Kolben mit seiner Stange, eine Öffnung des Zylinders an seinem hinteren Stirnende zum Durchtritt der Kolbenstange und eine Fassung am vorderen Stirnende des Zylinders zum Befestigen der Nadel aufweist (EP, A, 0 282 097).

Bei dieser Spritze ist die Nadel an einer Scheibe befestigt, die im vorderen Teil des Zylinders angeordnet ist und sich längs dessen Achse hin- und her verschieben läßt. Der hinter der Scheibe im Spritzenzylinder angeordnete Kolben, der starr mit seiner Kolbenstange verbunden ist, ist mit der Scheibe nicht verbunden, besitzt aber Greifer, die zum Kuppeln des Kolbens mit der Scheibe beim Zusammenwirken derselben an ihren Stirnflächen dienen. Bei Beendigung der Injektion wird der Kolben bei der Berührung mit der die Nadel tragenden Scheibe mit dieser gekoppelt, sodaß beim Versuch, die Spritze wiederholt mit dem Injektionsmittel zu füllen, die Scheibe samt der Nadel vom Kolben ins Innere des Zylinders eingezogen wird. Beim Eintritt der Nadel ins Innere des Zylinders kommt es zu einer Versetzung der Nadel in Bezug auf die Achse des Zylinders. Bei einem weiteren Versuch, eine wiederholte Injektion durchzuführen, kommt es zum Bruch der Injektionsnadel.

Bei dieser Ausführung besteht die Möglichkeit, die Spritze mehrmals zu verwenden, da der Kolben nur in seiner Endstellung im Vorderteil des Zylinders mit der Scheibe gekoppelt wird. Nur unter dieser Bedingung kommt es zum Einziehen der Nadel ins Innere des Zylinders, wodurch die Spritze für eine weitere Verwendung untauglich wird. Wenn aber der Kolben bei der Durchführung einer Injektion nicht in seine Endstellung geschoben wird, so kann die Spritze unter Verwendung fast des gesamten Inhaltes des Zylinders für eine beliebige Anzahl von Injektionen verwendet werden.

Offenbarung der Erfindung

Der Erfindung liegt die Aufgabe zugrunde, eine Einmalspritze für Injektionen zu entwickeln, bei der die Möglichkeit einer wiederholten Verwendung ausgeschlossen ist.

Die gestellte Aufgabe wird dadurch gelöst, daß bei einer Einmalspritze für Injektionen mit einem Zylinder, einem in diesem angeordneten Kolben mit einer Stange, einer Öffnung im hinteren Stirnende des Zylinders zum Durchtritt der Kolbenstange nach außen und einem Ansatz am vorderen Stirnende zur Befestigung der Injektionsnadel erfindungsgemäß die Kolbenstange profiliert in Gestalt einer Mehrzahl von in Richtung ihrer Länge aufeinanderfolgenden Kegelstümpfen ausgeführt ist, deren große und kleine Grundflächen einander jeweils gleich sind und die mit ihren kleinen Grundflächen zum Kolben gewandt sind, wobei im Zylinder konzentrisch zur Kolbenstange ein hohler profilierter Einsatz angeordnet ist, der die Kolbenstange umfaßt, aus wenigstens zwei längs der Achse der Kolbenstange voneinander trennbaren Teilen besteht, dessen Innenfläche der Außenfläche der Kolbenstange entspricht und dessen Außenfläche gebildet ist durch die Oberflächen einer Mehrzahl von längs der Kolbenstangenachse aufeinanderfolgenden und koaxial zu ihr liegenden Kegelstümpfen gebildet ist, deren große und kleine Grundflächen untereinander jeweils gleich sind und die mit ihren großen Grundflächen zum Kolben gewandt sind, und wobei der Durchmesser der Öffnung in der hinteren Stirnseite des Zylinders etwa gleich dem kleinsten Außendurchmesser des Einsatzes gewählt ist, und zwischen dem Kolben und dem Einsatz eine Ringscheibe konzentrisch zur Kolbenstange angeordnet ist, deren Öffnungsdurchmesser etwa dem kleinsten Durchmesser der Kolbenstange entspricht und deren seitliche Mantelfläche so abgeschrägt ist, daß der Durchmesser der zum Kolben gewandten Stirnfläche der Ringscheibe größer ist als der Durchmesser der zum Einsatz gewandten Stirnfläche der Ringscheibe, und wobei an der Innenfläche des Zylinders in der Nähe seiner hinteren Stirnseite ein Ringbund ausgebildet ist, dessen eine Oberfläche geneigt ist und der geneigten Oberfläche der Ringscheibe entspricht und dessen kleinster Durchmesser etwa dem kleinsten Außendurchmesser der Ringscheibe gleich ist.

Die Einmalspritze für Injektionen gemäß der vorliegenden Erfindung schließt die Möglichkeit einer wiederholten Verwendung für Injektionen aus, sie ist einfach in der Herstellung und zuverlässig im Gebrauch. Die Aufwendungen für die Herstellungen der Einmalspritzen gemäß der Erfindung sind praktisch nicht höher als die Aufwendungen zur Herstellung von zur Zeit bekannten Einmalspritzen.

Kurzbeschreibung der Zeichnungen

Die Erfindung wird nachfolgend durch die Beschreibung eines konkreten Ausführungsbeispiels an Hand der beigegebenen Zeichnungen erläutert. In diesen zeigt:

Fig. 1, 2, 3, 4, 5 (a, b) eine Einmalspritze für Injektionen gemäß der Erfindung im auseinandergenommenen Zustand in isometrischer Darstellung;

Fig. 6 die Einmalspritze für Injektionen in der Ausgangsstellung, Längsschnitt;

Fig. 7 die Einmalspritze für Injektionen nach dem Aufziehen des Injektionsmittels, teilweiser Längsschnitt in isometrischer Darstellung;

Fig. 8 das Gleiche wie in Fig. 6 bei der Durchführung einer Injektion.

Beste Ausführungsform der Erfindung

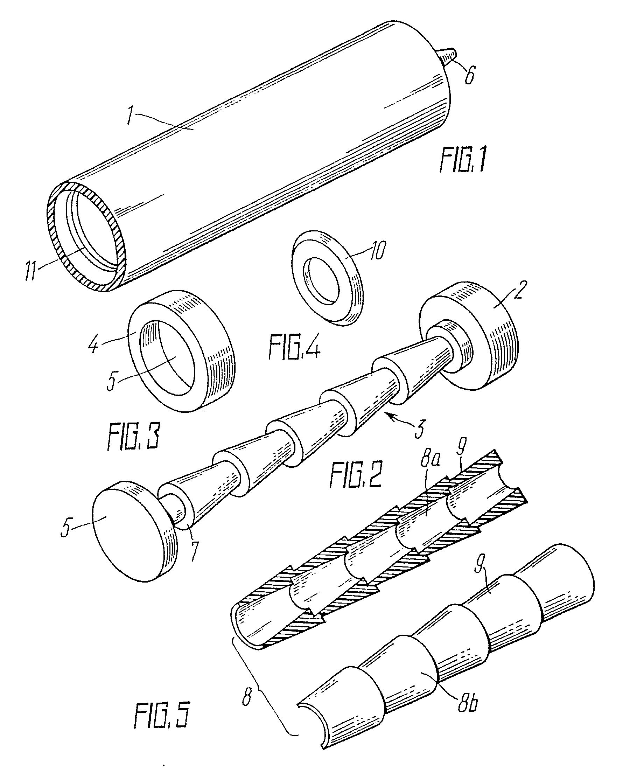

Die Einmalspritze für Injektionen gemäß der Erfindung besteht aus einem Zylinder 1 (Fig. 1), einem in diesem angeordneten Kolben 2 (Fig. 2, 6) mit einer Kolbenstange 3. Der Zylinder 1 hat an seiner hinteren Stirnseite einen unlösbar an ihm befestigten Deckel 4 (Fig. 3, 6), in dem eine zentrale Öffnung 5 für den Durchtritt der Kolbenstange 3 ausgeführt ist. Am vorderen Stirnende des Zylinders 1 ist ein Ansatz 6 zur Befestigung einer Injektionsnadel - Kanüle - ausgebildet (Fig. 1, 6).

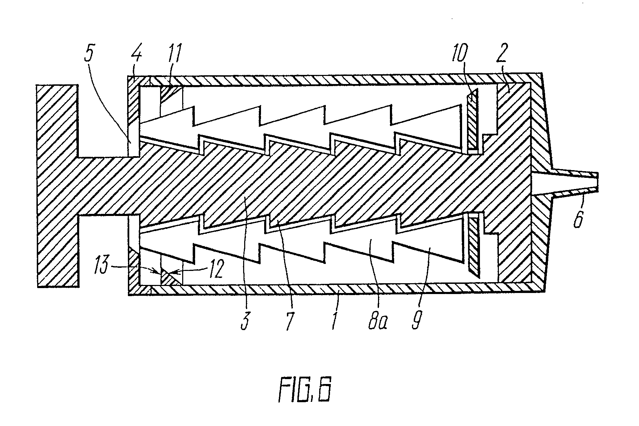

Die Kolbenstange 3 (Fig. 2, 6) ist profiliert ausgebildet, und zwar in Gestalt einer Vielzahl von längs der Kolbenstange 3 aufeinanderfolgenden Kegelstümpfen 7, deren große Grundflächen einander gleich sind und die mit ihren ebenfalls einander gleichen kleinen Grundflächen zum Kolben 2 gewandt sind. Im Zylinder 1 ist konzentrisch zur Kolbenstange 3 ein hohler Profileinsatz 8 (Fig. 5 (a, b), 6), der längs der Achse der Kolbenstange 3 teilbar ist und aus wenigstens zwei, im beschriebenen Ausführungsbeispiel aus zwei Teilen 8a, 8b besteht, die die Kolbenstange 3 umfassen und der eine innere Oberfläche hat, die der Oberfläche der Kolbenstange 3 entspricht. Die Außenfläche des Einsatzes 8 ist gebildet von den Oberflächen von Kegelstümpfen, die längs der Achse der Kolbenstange 3 aufeinanderfolgend und koaxial zu ihr angeordnet sind, deren kleine Grundflächen einander gleich sind und deren ebenfalls einander gleichen großen Grundflächen zum Kolben 2 gewandt sind.

Der Durchmesser der Öffnung 5 zum Durchtritt der Kolbenstange 3 im Deckel 4 des Gehäuses 1 ist etwa gleich dem kleinsten Außendurchmesser des Einsatzes 8 gewählt. Zwischen dem Kolben 2 und dem zum Kolben 2 gewandten Stirnende des Einsatzes 8 ist konzentrisch zur Kolbenstange 3 eine Ringscheibe 10 (Fig. 4, 6) angeordnet, deren Innendurchmesser etwa gleich dem kleinsten Durchmesser der Kolbenstange 3 ist. Die äußere Mantelfläche der Ringscheibe 10 ist derart geneigt, daß der Durchmesser der zum Kolben 2 gelegenen Stirnfläche der Scheibe 10 größer ist als der Durchmesser der zum Einsatz 8 gelegenen Stirnfläche der Scheibe 10.

An der Innenfläche des Gehäuses 1 der Spritze ist in der Nähe ihres hinteren Stirnendes mit der Öffnung 5 ein Ringbund 11 dreieckigen Profils ausgebildet, dessen Oberfläche 12 unter einem Winkel von weniger als 90° zur Seitenfläche des Zylinders 1 geneigt ist. Der kleinste Durchmesser des Ringbundes 11 ist ungefähr gleich dem kleinsten Außendurchmesser der Ringscheibe 10. Die geneigte Oberfläche 12 entspricht der geneigten Mantelfläche der Scheibe 10 und wirkt mit dieser zusammen. Die Oberfläche 13 des Ringbundes 11 verläuft parallel zur Stirnfläche des Gehäuses 1. Der Abstand zwischen der Oberfläche 13 des Ringbundes 11 und der Innenfläche des Deckels 4 ist gleich oder größer als die Dicke der Scheibe 10.

Die Spritze ist aus einem elastischen Polymerwerkstoff hergestellt.

Man gebraucht die Spritze auf die folgende Weise:

Zum Aufziehen des Injektionsmittels wird die Kolbenstange 3 aus dem Zylinder 1 der Spritze (Fig. 6) herausgezogen. Dabei treten die Kegelstümpfe 9 des konzentrisch zur Kolbenstange 3 liegenden Einsatzes 8 mit einem geringen Widerstand durch die zum Durchtritt der Kolbenstange 3 vorgesehene Öffnung 5 im Deckel 4 des Zylinders 1 der Spritze. Diese Bewegung ist ermöglicht durch die Form der Kegelstümpfe 9 des Einsatzes 8, deren kleinster Durchmesser etwa gleich dem Durchmesser der Öffnung 5 im Deckel 4 des Zylinders 1 der Spritze ist, sowie der Elastizität des Polymerwerkstoffs, aus dem die Spritze hergestellt ist.

In der Gegenrichtung ist eine Verschiebung des Kolbens 3 * nicht möglich, da der Durchmesser der großen Grundflächen der Kegelstümpfe 9 des Einsatzes 8 größer ist als der Durchmesser der Öffnung 5 im Deckel 4 des Zylinders 1 der Spritze und das Profil der Außenfläche des Einsatzes 8 dies ebenfalls verhindert. Bei Beendigung des Aufziehens des Injektionsmittels wirkt die Scheibe 10 mit ihrer äußeren Mantelfläche zusammen mit der kegeligen Oberfläche 12 des Ringbundes 11 an der Innenfläche des Zylinders 1 der Spritze, gelangt hinter diesen Ringbund 11 und bleibt in dem Raum zwischen der Innenfläche des Deckels 4 des Zylinders 1 der Spritze und der Oberfläche 13 des Ringbundes 11 stehen. Nach dem Übertritt der Scheibe 10 hinter den Ringbund 11 wird seine Rückbewegung unmöglich, da der Durchmesser der Stirnfläche der Scheibe 10, die in dieser Lage derselben dem Ringbund 11 zugewandt ist, größer ist als der Durchmesser der vom Ringbund 11 umgrenzten Öffnung.

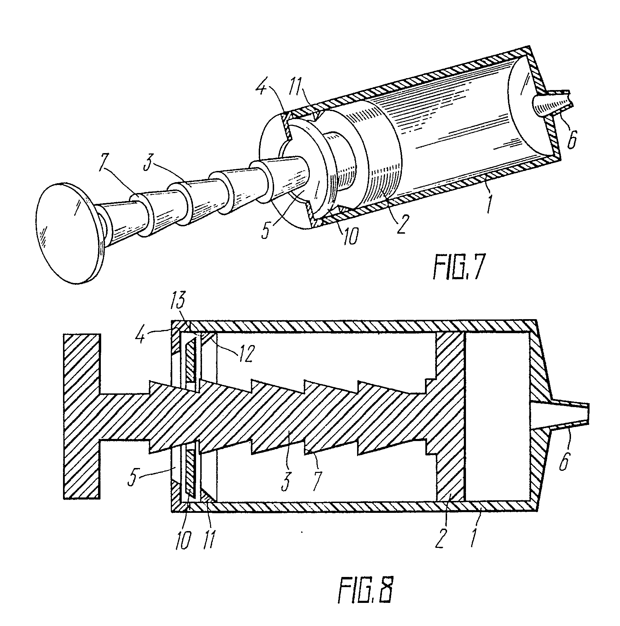

Wenn die Scheibe 10 in den Raum zwischen dem Deckel 4 und dem Ringbund 11 eingetreten ist, befindet sich der Einsatz 8 außerhalb des Zylinders 1 und seine Teile 8a und 8b können sich ohne Widerstand von der Kolbenstange 3 lösen und fallen weg (Fig. 7).

Zur Verabreichung des Injektionsmittels wird die Kolbenstange 3 in Richtung zum vorderen Stirnende des Gehäuses 1 (Fig. 8) der Spritze verschoben. Dabei treten die Kegelstümpfe 7 der Kolbenstange 3 mit einem geringen Widerstand durch die zentrale Öffnung der Scheibe 10, deren Durchmesser ungefähr gleich dem kleinsten Durchmesser der Kolbenstange 3 ist. Eine entgegengesetzte Bewegung der Kolbenstange 3 ist nicht möglich, da der Durchmesser der großen Grundflächen der Kegelstümpfe 7 der Kolbenstange 3 größer ist als der Durchmesser der Öffnung der Scheibe 10.

Auf diese Weise ist die Möglichkeit einer wiederholten Verwendung der Einmalspritze für Injektionen ausgeschlossen.

Nach Beendigung der Injektion ist die Spritze zur nochmaligen Verwendung unbrauchbar geworden und ist zu verwerten.

Eine weitgehende Anwendung der vorgeschlagenen Spritze kann praktisch ausschließen, daß Patienten bei Injektionen mit dem AIDS-Virus, einer infektiösen Hepatitis oder anderen Krankheiten angesteckt werden, die bei einer parenteralen Verabreichung von Arzneimitteln übertragen werden.

Industrielle Anwendbarkeit

Die vorgeschlagene Spritze für Injektionen kann in beliebigen medizinischen Einrichtungen sowie beim privaten Gebrauch verwendet werden.

Ansprüche

1. Einmalspritze für Injektionen mit einem Zylinder (1), einem in diesem angeordneten Kolben (2) mit einer Stange (3), einer Öffnung (5) im hinteren Stirnende des Zylinders (1) zum Durchtritt der Kolbenstange (3) nach außen und einem Ansatz am vorderen Stirnende zur Befestigung der Injektionsnadel,
dadurch gekennzeichnet, daß die Kolbenstange (3) profiliert in Gestalt einer Mehrzahl von in Richtung ihrer Länge aufeinanderfolgenden Kegelstümpfen (7) ausgeführt ist, deren große und kleine Grundflächen einander jeweils gleich sind und die mit ihren kleinen Grundflächen zum Kolben (2) gewandt sind, daß im Zylinder (1) konzentrisch zur Kolbenstange (3) ein hohler profilierter Einsatz (8) angeordnet ist, der die Kolbenstange (3) umfaßt, aus wenigstens zwei längs der Achse der Kolbenstange (3) voneinander getrennten Teilen (8a, 8b) besteht, dessen Innenfläche der Außenfläche der Kolbenstange (3) entspricht und dessen Außenfläche durch die Oberflächen einer Mehrzahl von längs der Kolbenstangenachse aufeinanderfolgenden und koaxial zu ihr liegenden Kegelstümpfen (9) gebildet ist, deren große und kleine Grundflächen untereinander jeweils gleich sind und die mit ihrer großen Grundfläche zum Kolben (2) gewandt sind, wobei der Durchmesser der Öffnung (5) in der hinteren Stirnseite des Zylinders (1) etwa gleich dem kleinsten Außendurchmesser des Einsatzes (8) gewählt ist, daß zwischen dem Kolben (2) und dem Einsatz (8) eine Ringscheibe (10) konzentrisch zur Kolbenstange (3) angeordnet ist, deren Öffnungsdurchmesser

* im Ursprungstext enthaltener Fehler; richtig ″des Kolbens 2″

etwa dem kleinsten Außendurchmesser der Kolbenstange (3) entspricht und deren seitliche Mantelfläche so abgeschrägt ist, daß der Durchmesser der zum Kolben (2) gewandten Stirnfläche der Ringscheibe (10) größer ist als der Durchmesser der zum Einsatz (8) gewandten Stirnfläche der Ringscheibe (10), und daß an der Innenfläche des Zylinders (1) in der Nähe von dessen hinterer Stirnseite ein Ringbund (11) ausgebildet ist, dessen eine Oberfläche (12) geneigt ausgebildet ist, der geneigten Mantelfläche der Ringscheibe (10) entspricht und dessen kleinster Durchmesser etwa dem kleinsten Außendurchmesser der Ringscheibe (10) gleich ist.

1

FIG.1

6

10

FIG.4

2

4

5

FIG.3

3

5

7

FIG.2

8a

9

8

9

8b

FIG.5

11

FIG. 6

FIG. 7

FIG. 8

# INTERNATIONAL SEARCH REPORT

International Application No PCT/SU 89/00266

**I. CLASSIFICATION OF SUBJECT MATTER** (If several classification symbols apply, indicate all) [6]

According to International Patent Classification (IPC) or to both National Classification and IPC

IPC[5]: A 61M 5/50

**II. FIELDS SEARCHED**

Minimum Documentation Searched [7]

| Classification System | Classification Symbols |
|---|---|
| IPC[4] | A6IM 5/00, 5/18, 5/24, 5/28, 5/31 |

Documentation Searched other than Minimum Documentation
to the Extent that such Documents are Included in the Fields Searched [8]

**III. DOCUMENTS CONSIDERED TO BE RELEVANT** [9]

| Category [*] | Citation of Document, [11] with indication, where appropriate, of the relevant passages [12] | Relevant to Claim No. [13] |
|---|---|---|
| A | AU, B, 16859 (JONS HESER DEAX), 12 December 1934 (12.12.34), the claims, figures 1,2 | 1 |
| A | FR, AI, 2298340 (BLANIE MARIE JEAN MICHEL PAUL), 20 August 1976 (20.08.76), the claims, figure 1 | 1 |
| A | US, A, 4775363 (CHRISTIAN SANDSDALEN), 4 October 1988 (04.10.88), the claims, the drawing | 1 |

* Special categories of cited documents: [10]

"A" document defining the general state of the art which is not considered to be of particular relevance

"E" earlier document but published on or after the international filing date

"L" document which may throw doubts on priority claim(s) or which is cited to establish the publication date of another citation or other special reason (as specified)

"O" document referring to an oral disclosure, use, exhibition or other means

"P" document published prior to the international filing date but later than the priority date claimed

"T" later document published after the international filing date or priority date and not in conflict with the application but cited to understand the principle or theory underlying the invention

"X" document of particular relevance; the claimed invention cannot be considered novel or cannot be considered to involve an inventive step

"Y" document of particular relevance; the claimed invention cannot be considered to involve an inventive step when the document is combined with one or more other such documents, such combination being obvious to a person skilled in the art.

"&" document member of the same patent family

**IV. CERTIFICATION**

| Date of the Actual Completion of the International Search | Date of Mailing of this International Search Report |
|---|---|
| 27 February 1990 (27.02.90) | 06 April 1990 (06.04.90) |

| International Searching Authority | Signature of Authorized Officer |
|---|---|
| ISA/SU | |

Form PCT/ISA/210 (second sheet) (January 1985)